# EUROPÄISCHE PATENTANMELDUNG

(11) **EP 3 714 813 A1**
(43) Veröffentlichungstag der Anmeldung: **30.09.2020**
(21) Anmeldenummer: 19166244.4
(22) Anmeldetag: 29.03.2019
(51) Int. Cl.: A61B 17/16

(54) **CHIRURGISCHES INSTRUMENT**

(71) Anmelder: Gebrüder Zepf Medizintechnik GmbH & Co. KG, 78589 Dürbheim (DE)
(72) Erfinder: Zepf, Christoph, 78589 Dürbheim (DE); Maier, Franz, 78559 Gosheim (DE)
(74) Vertreter: Durm Patentanwälte PartG mbB

(57) **Zusammenfassung**

Die vorliegende Erfindung betrifft ein chirurgisches Instrument (10) mit: einem Hauptteil (18) mit einem feststehenden Maulteil (24) an einem Ende; einem Schieber (16), der relativ zu dem Hauptteil entlang einer Längsachse (20) verschiebbar gelagert ist; und einem beweglichen Maulteil (22) zum Ausführen einer Drehbewegung gegenüber dem feststehenden Maulteil und dem Schieber zwischen einer geöffneten Position und einer geschlossenen Position, um mit dem feststehenden Maulteil zum Greifen und/oder Schneiden von Gewebe zusammenzuwirken, wobei das Hauptteil ein erstes bogenförmiges Führungselement (38) umfasst und der Schieber ein zweites bogenförmiges Führungselement (42) umfasst; das bewegliche Maulteil zum Ausführen der Drehbewegung ein erstes bogenförmiges Steuerungselement (28) umfasst, das mit dem ersten Führungselement in einer Kulissensteuerung zusammenwirkt, und ein zweites bogenförmiges Steuerungselement (30) umfasst, das mit dem zweiten Führungselement in einer Kulissensteuerung zusammenwirkt; und eine Krümmungsrichtung des ersten Führungselements einer Krümmungsrichtung des zweiten Führungselements entspricht. Die vorliegende Erfindung betrifft weiterhin ein Verfahren zum Herstellen eines chirurgischen Instruments (10).

## Beschreibung

Die vorliegende Erfindung betrifft ein chirurgisches Instrument, insbesondere einen Rongeur, zum Greifen und/oder Schneiden von Gewebe.

Rongeure werden eingesetzt, um Gewebe zu entfernen bzw. Gewebe zu greifen. Zumeist ist die Spitze des Instruments ausgehöhlt oder abgekantet und hat scharfkantige Schneiden. Anwendungen liegen insbesondere im Bereich der Rückenchirurgie (sog. Conchotome bzw. Intervertebral Disc (IVD) Rongeure), aber auch in anderen Bereichen, in denen Knochen- und anderes Gewebe an schwer zugänglichen Stellen abgetragen werden muss.

Derartige chirurgische Instrumente umfassen zumeist einen länglichen Schaft, der im Wesentlichen aus einem Hauptteil und einem beweglichen Teil (Schieber) besteht. An einem Arbeitsende des Schafts ist ein bewegliches Maulteil angeordnet, das gegenüber einem feststehenden Maulteil eine Rotationsbewegung ausführen kann, um hierdurch Gewebe zwischen feststehendem und beweglichem Maulteil zu greifen und/oder zu schneiden.

Auf der einen Seite können bei der Verwendung derartiger chirurgischer Instrumente gerade dann, wenn Knochengewebe abgetragen werden soll, vergleichsweise hohe Kräfte auftreten, sodass eine stabile Bauweise erforderlich ist. Auf der anderen Seite ist es im Sinne einer möglichst minimal invasiven Anwendung vorteilhaft, wenn das chirurgische Instrument möglichst filigran ausgeführt wird, um eine Schädigung umliegenden Gewebes zu minimieren. Dieses Abwägen zwischen filigraner Bauweise einerseits und hoher Belastbarkeit andererseits ist insbesondere im Bereich des Arbeitsendes des Instruments relevant, wo üblicherweise eine Kopplung zueinander beweglicher Komponenten notwendig ist, um die Greif- und Schneidbewegung auszuführen.

Die Kopplung bzw. die gelenkartige Verbindung wird im Stand der Technik oftmals durch Drehgelenke gelöst, bei denen ein Stift zur Verbindung zweier zueinander drehbarer Teile eingesetzt wird. Ein anderer Ansatz liegt in der Verwendung von Kurvengelenken bzw. Kulissenführungen, bei denen zueinander bewegliche Teile in Form einer Zwangssteuerung geführt werden. Drehgelenke haben den Nachteil, dass eine hohe Kraft auf den Stift einwirkt, sodass dieser sowie die den Stift umgebenden Materialbereiche stark belastet werden und brechen können. Kulissensteuerungen bzw. Kurvengelenke haben den Nachteil, dass sie einen vergleichsweise hohen Bauraumbedarf bedingen, wodurch eine filigrane Realisierung des Arbeitsendes des chirurgischen Instruments erschwert wird.

In diesem Zusammenhang wird in US 4,712,545 ein chirurgisches Instrument mit einem ersten stationären Maulteil und einem zweiten beweglichen Maulteil, das zum Ausführen einer Drehbewegung gegenüber dem stationären Maulteil ausgebildet ist, offenbart. Das bewegliche Maulteil ist mit dem stationären Maulteil durch eine erste gebogene Vorsprung- und Nutverbindung verbunden. Das bewegliche Maulteil ist zudem über eine zweite Vorsprung- und Nutverbindung mit einem Koppelteil verbunden. Wenn das Koppelteil zu dem stationären Maulteil in eine erste Richtung bewegt wird, öffnet sich das bewegliche Maulteil weg vom stationären Maulteil. Wenn das Koppelelement in eine Gegenrichtung bewegt wird, schließt sich das bewegliche Maulteil in Richtung des stationären Maulteils. Ein Nachteil der Konstruktion liegt in einem vergleichsweise großen Bauraumbedarf. Weiterhin ist die Montage vergleichsweise komplex, da aufgrund der Kopplung des beweglichen Maulteils mit dem stationären Maulteil und dem Koppelelement ein Einsetzen des beweglichen Maulteils durch eine Öffnung im stationären Maulteil hindurch erfolgen muss.

Weiterhin wird in US 7,559,940 B2 ein chirurgisches Stanzwerkzeug offenbart. Das Stanzwerkzeug umfasst ein oberes Maulteil und ein unteres Maulteil, die an ihren körperabgewandten Enden verbunden sind. Die Maulteile haben Schneidekanten an ihren körperzugewandten Enden. Ein Drehpunkt der Schnitttrajektorie kann sich oberhalb des initialen Schnittpunkts des unteren Maulteils befinden. Das obere Maulteil kann eine umgekehrte Schneidekante umfassen. Die Maulteile können gegenüber einander verschiebbar gekoppelt sein. Das Koppelelement kann einen Stift sowie einen Schlitz umfassen, um das bewegliche Maulteil mit einem Steuerungshebel zu verbinden. Nachteilig an dieser Konstruktion ist einerseits die Anfälligkeit der Stiftverbindung aufgrund hoher wirkender Kräfte und andererseits die vergleichsweise komplexe Montage.

Ausgehend hiervon stellt sich der vorliegenden Erfindung die Aufgabe, ein stabiles chirurgisches Instrument zu schaffen, das eine filigrane Ausführung erlaubt. Insbesondere soll eine Kopplung eines beweglichen Maulteils an ein feststehendes Maulteil und einen Schieber mit einer hohen Widerstandsfähigkeit gegenüber einem Ausbrechen oder anderen Defekten bereitgestellt werden. Zudem soll eine effiziente Montage unabhängig von der Geometrie des Maulteils ermöglicht werden.

Zum Lösen dieser Aufgabe betrifft ein Aspekt der vorliegenden Erfindung ein chirurgisches Instrument mit:
- einem Hauptteil mit einem feststehenden Maulteil an einem Ende;
- einem Schieber, der relativ zu dem Hauptteil entlang einer Längsachse verschiebbar gelagert ist; und
- einem beweglichen Maulteil zum Ausführen einer Drehbewegung gegenüber dem feststehenden Maulteil und dem Schieber zwischen einer geöffneten Position und einer geschlossenen Position, um mit dem feststehenden Maulteil zum Greifen und/oder Schneiden von Gewebe zusammenzuwirken, wobei
- das Hauptteil ein erstes bogenförmiges Führungselement umfasst und der Schieber ein zweites bogenförmiges Führungselement umfasst;
- das bewegliche Maulteil zum Ausführen der Drehbewegung ein erstes bogenförmiges Steuerungselement umfasst, das mit dem ersten Führungselement in einer Kulissensteuerung zusammenwirkt, und ein zweites bogenförmiges Steuerungselement umfasst, das mit dem zweiten Führungselement in einer Kulissensteuerung zusammenwirkt; und
- eine Krümmungsrichtung des ersten Führungselements einer Krümmungsrichtung des zweiten Führungselements entspricht.

In einem weiteren Aspekt betrifft die vorliegende Erfindung ein Verfahren zum Herstellen eines chirurgischen Instruments, mit einem Hauptteil mit einem feststehenden Maulteil an einem Ende; einem Schieber, der relativ zu dem Hauptteil entlang einer Längsachse verschiebbar gelagert ist; und einem beweglichen Maulteil zum Ausführen einer Drehbewegung gegenüber dem feststehenden Maulteil und dem Schieber zwischen einer geöffneten Position und einer geschlossenen Position, um mit dem feststehenden Maulteil zum Greifen und/oder Schneiden von Gewebe zusammenzuwirken, mit den Schritten:
- Fräsen eines ersten bogenförmigen Führungselements in das Hauptteil und eines zweiten bogenförmigen Führungselements in den Schieber;
- Fräsen eines ersten bogenförmigen Steuerungselements zum Ausführen der Drehbewegung in das bewegliche Maulteil, das mit dem ersten Führungselement in einer Kulissensteuerung zusammenwirkt, und eines zweiten bogenförmigen Steuerungselements in das bewegliche Maulteil, das mit dem zweiten Führungselement in einer Kulissensteuerung zusammenwirkt, wobei
- eine Krümmungsrichtung des ersten Führungselements einer Krümmungsrichtung des zweiten Führungselements entspricht.

Bevorzugte Ausgestaltungen der Erfindung werden in den abhängigen Ansprüchen beschrieben. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Insbesondere kann das Verfahren entsprechend der für das chirurgische Instrument in den abhängigen Ansprüchen beschriebenen Ausgestaltungen ausgeführt sein.

Erfindungsgemäß wird zum Ausführen einer Drehbewegung des beweglichen Maulteils gegenüber dem feststehenden Maulteil eine Kulissensteuerung verwendet. Das bewegliche Maulteil wird sowohl in Relation zu dem Hauptteil als auch in Relation zu dem Schieber in einer Zwangsführung bewegt bzw. gedreht. Die Steuerungselemente am beweglichen Maulteil führen relativ zu den Führungselementen am Hauptteil und am Schieber eine Gleit- und/oder Wälzbewegung aus. Die Steuerungselemente können nur die durch die Führungselemente vorgegebene Bewegung ausführen. Insoweit entspricht die Kulissensteuerung einem Kurvengelenk bzw. einer Zwangssteuerung. Durch eine Längsverschiebung des Schiebers relativ zu dem Hauptteil wirken die Kopplung des beweglichen Maulteils mit dem Hauptteil und die Kopplung des beweglichen Maulteils mit dem Schieber derart zusammen, dass das bewegliche Maulteil eine Drehbewegung gegenüber dem feststehenden Maulteil und dem Schieber ausführt. Hierdurch kann das Maulteil basierend auf der Bewegung von Hauptteil und Schieber geöffnet bzw. geschlossen werden. Wenn das feststehende Maulteil und das bewegliche Maulteil in Kontakt stehen, ist die geschlossene Position erreicht. Beim Schließen der Maulteile kann Gewebe geschnitten oder gegriffen werden.

Im Vergleich zu bisherigen Ansätzen, bei denen eine Verbindung der zueinander beweglichen Teile über einen Stift bzw. einen Pin realisiert wird, bietet der erfindungsgemäße Ansatz eine höhere Stabilität. Ein Ausbrechen an der Verbindung zwischen beweglichem Maulteil und Hauptteil bzw. Schieber bzw. ein Brechen des Stifts werden vermieden. Bei gleicher Baugröße kann eine stabilere Ausführung erreicht werden. Bei gleicher Stabilität ist eine kleinere Baugröße möglich. Im Vergleich zu bisherigen Ansätzen mit einer Kulissenführung mit gegenläufigen Krümmungsrichtungen der bogenförmigen Führungselemente erlaubt das erfindungsgemäße chirurgische Instrument eine wesentlich vereinfachte Montage. Insbesondere wird es möglich, aufgrund der gleichgerichteten bogenförmigen Führungselemente das bewegliche Maulteil bei der Montage von einer Oberseite aus einzusetzen. Insoweit eignet sich der erfindungsgemäße Ansatz dazu, chirurgische Instrumente zu realisieren, bei denen weder das feststehende Maulteil noch das bewegliche Maulteil eine Öffnung aufweisen. Es wird eine einfache Montage ermöglicht.

In einer bevorzugten Ausgestaltung umfasst das erste Führungselement einen bogenförmigen Vorsprung und das erste Steuerungselement eine bogenförmige Nut, in die der Vorsprung eingreift. Alternativ oder zusätzlich umfasst das zweite Führungselement einen bogenförmigen Vorsprung und das zweite Steuerungselement eine bogenförmige Nut, in die der Vorsprung eingreift. Es ist also zumindest eines der Führungselemente als Vorsprung ausgebildet, der mit einem als Nut ausgebildeten Steuerungselement zusammenwirkt. Dadurch, dass am beweglichen Maulteil Nuten und am feststehenden Maulteil bzw. am Schieber Vorsprünge vorgesehen sind, ergibt sich eine vereinfachte Fertigbarkeit. Die Kosten bei der Herstellung können reduziert werden.

In einer weiteren vorteilhaften Ausgestaltung sind das erste Führungselement und das erste Steuerungselement kreisbogenförmig mit identischem Radius ausgebildet. Zusätzlich oder alternativ sind das zweite Führungselement und das zweite Steuerungselement kreisbogenförmig mit identischem Radius ausgebildet. Durch die Verwendung eines Kreisbogens kann eine kreisförmige Bewegung bzw. eine Drehung um einen feststehenden Drehpunkt erreicht werden. Eine kreisförmige Drehung erfolgt dabei einerseits zwischen Schieber und beweglichem Maulteil und andererseits zwischen feststehendem Maulteil bzw. Hauptteil und beweglichem Maulteil. Ein identischer Radius bei beiden Kopplungen bewirkt eine effiziente Herstellbarkeit. Zudem bewirkt ein identischer Radius eine gleichförmige Krafteinwirkung, wodurch eine optimale Stabilität erreicht wird.

In einer vorteilhaften Ausgestaltung entspricht ein Kreisbogenradius des ersten Führungselements einem Kreisbogenradius des zweiten Führungselements. Durch die Verwendung identischer Radien wird ein optimaler Ausgleich bezüglich der Krafteinwirkung auf die Bauteile erreicht. Hieraus ergibt sich eine optimale Stabilität des chirurgischen Instruments.

In einer vorteilhaften Ausgestaltung entspricht ein Mittelpunktswinkel eines Kreisbogens des ersten Führungselements einem Mittelpunktswinkel eines Kreisbogens des ersten Steuerungselements. Zusätzlich oder alternativ entspricht ein Mittelpunktswinkel eines Kreisbogens des zweiten Führungselements einem Mittelpunktswinkel eines Kreisbogens des zweiten Steuerungselements. Dadurch, dass die gleichen Mittelpunktswinkel verwendet werden, entsprechen sich die Führungselemente und die Steuerungselemente in ihrer Länge. Die Länge der Überdeckung des Führungselements mit dem Steuerungselement bedingt die Länge, auf der die Kraft beim Betätigen des chirurgischen Instruments einwirkt. Durch die Verwendung identischer Mittelpunktswinkel kann eine große Länge der Krafteinwirkung realisiert werden, wodurch die Stabilität verbessert wird.

In einer vorteilhaften Ausgestaltung liegt ein Mittelpunktswinkel eines Kreisbogens des ersten Führungselements zwischen 160° und 180°. Zusätzlich oder alternativ liegt ein Mittelpunktswinkel eines Kreisbogens des zweiten Führungselements zwischen 160° und 180°. Dies bewirkt einen großen Bewegungsspielraum. Es wird ein weites Öffnen des beweglichen Maulteils in Bezug auf das feststehende Maulteil erreicht. Eine weite Öffnung erlaubt ein breites Anwendungsspektrum.

In einer vorteilhaften Ausgestaltung umfasst das erste Steuerungselement zwei bogenförmige erste Teil-Steuerungselemente, die bezüglich einer durch die Längsachse des Hauptteils verlaufenden Bewegungsebene des beweglichen Maulteils symmetrisch auf zwei Außenseiten des beweglichen Maulteils angeordnet sind und mit zwei bogenförmigen ersten Teil-Führungselementen des ersten Führungselements an Innenseiten eines Ausschnitts im Hauptteil zusammenwirken. Zusätzlich oder alternativ umfasst das zweite Steuerungselement in entsprechender Weise zwei bogenförmige zweite Teil-Steuerungselemente, die bezüglich einer durch die Längsachse des Hauptteils verlaufenden Bewegungsebene des beweglichen Maulteils symmetrisch auf zwei Außenseiten des beweglichen Maulteils angeordnet sind und mit zwei bogenförmigen zweiten Teil-Führungselementen des zweiten Führungselements an Innenseiten eines Ausschnitts im Schieber zusammenwirken. Durch die Verwendung symmetrisch angeordneter Teil-Führungselemente, die mit Teil-Steuerungselementen zusammenwirken, kann die einwirkende Kraft bei der Betätigung des chirurgischen Instruments auf zwei Seiten des beweglichen Maulteils verteilt werden. Die Verwendung zweier Teil-Führungs- bzw. Teil-Steuerungselemente bewirkt insoweit eine verbesserte Stabilität. Eine symmetrische Krafteinwirkung beidseitig des beweglichen Maulteils erlaubt bei gleicher Stabilität einen kleineren Bauraum und führt insoweit zu einer filigraneren Ausführung des chirurgischen Instruments. Die Verwendung außenliegender Steuerungselemente am beweglichen Maulteil bzw. innenliegender Führungselemente am Hauptteil und am Schieber erlaubt eine effiziente Herstellbarkeit der einzelnen Elemente sowie eine einfache Montage.

In einer vorteilhaften Ausgestaltung sind das bewegliche Maulteil und die beiden Steuerungselemente einstückig als Frästeil ausgebildet. Zusätzlich oder alternativ sind das Hauptteil und das erste Führungselement einstückig als Frästeil ausgebildet. Zusätzlich oder alternativ sind der Schieber und das zweite Führungselement einstückig als Frästeil ausgebildet. Dadurch, dass das bewegliche Maulteil, das Hauptteil sowie der Schieber jeweils mit den zugeordneten Steuerungs- bzw. Führungselementen einstückig ausgebildet sind, kann eine effiziente Fertigbarkeit erreicht werden. Die Teile werden jeweils in einem Fräsvorgang hergestellt. Insbesondere können hierzu CNC- oder NC-Fräsen eingesetzt werden. Eine präzise und gleichzeitig kosteneffiziente Fertigung wird erreicht.

In einer bevorzugten Ausgestaltung sind das bewegliche Maulteil und/oder das feststehende Maulteil löffelförmig ausgebildet. Unter löffelförmig versteht sich dabei insbesondere, dass das jeweilige Maulteil keine durchgängige Öffnung bzw. keinen Ausschnitt aufweist. Das chirurgische Instrument ist insoweit als Conchotom bzw. IVD-Rongeur ausgebildet.

In einer vorteilhaften Ausgestaltung verringert sich ein Querschnitt des Hauptteils, des Schiebers und des beweglichen Maulteils senkrecht zu der Längsachse des Hauptteils in Richtung des feststehenden Maulteils. Durch eine Verjüngung des chirurgischen Instruments in Richtung des Arbeitsendes (in geschlossenem Zustand), das innerhalb des menschlichen Körpers eingesetzt wird, wird eine verbesserte Anwendbarkeit erreicht. Beim Herausziehen des Instruments werden Verletzungen vermieden. Zudem ergeben sich Vorteile in Bezug auf das Handling durch den Chirurgen.

In einer bevorzugten Ausgestaltung liegt ein Drehpunkt der Drehbewegung des beweglichen Maulteils gegenüber dem Schieber außerhalb des beweglichen Maulteils. Dadurch, dass ein Drehpunkt außerhalb des beweglichen Maulteils vorgesehen wird, kann ein vergleichsweise großer Bewegungsumfang realisiert werden. Eine Anwendung in unterschiedlichen Anwendungsbereichen wird möglich.

In einer vorteilhaften Ausgestaltung liegt ein Drehpunkt der Drehbewegung des beweglichen Maulteils gegenüber dem festen Maulteil innerhalb des beweglichen Maulteils. Durch diese Lage des Drehpunkts kann eine hohe Stabilität erreicht werden.

In einer weiteren Ausgestaltung weist die geöffnete Position des beweglichen Maulteils einen Öffnungswinkel von 45° bis 55° auf. Durch einen derart großen Öffnungswinkel zwischen beweglichem Maulteil und feststehendem Maulteil können verschiedene Anwendungen umgesetzt werden. Insbesondere wird es möglich, größere Gewebestücke abzuschneiden bzw. abzutragen.

In einer vorteilhaften Ausgestaltung sind das bewegliche Maulteil sowie das feststehende Maulteil aus Edelstahl ausgebildet. Edelstahl als biokompatibler Werkstoff vermindert körperliche Reaktionen der mit dem chirurgischen Instrument operierten Person. Eine möglichst komplikationsfreie Anwendung wird erreicht.

Unter einer Drehbewegung versteht sich hierin eine Bewegung entlang einer Kurve. Eine Drehbewegung muss nicht zwangsweise um einen feststehenden Drehpunkt erfolgen, der Drehpunkt kann sich sozusagen während der Drehung bewegen. Unter einem bogenförmigen Element versteht sich ein Element, das im Sinne einer Kurve gebogen bzw. gekrümmt ist. Der Bogen verläuft dabei in einer zweidimensionalen Ebene und weist vorzugsweise einen sprungfreien Verlauf auf. Unter einer Krümmungsrichtung wird ein Verlauf des Bogens in einer zweidimensionalen Ebene, die den Bogen umfasst, verstanden. Ein Bogen ist hierin nicht zwangsweise als Kreisbogen zu verstehen. In einer Kulissensteuerung wirken zwei Elemente zusammen und werden in Bezug zueinander zwangsgeführt. Eine Kulissensteuerung kann auch als Zwangsführung bzw. als Kurvengelenk bezeichnet werden. Insbesondere kann eine wälzende oder gleitende Führung verwendet werden.

Die Erfindung wird nachfolgend anhand einiger ausgewählter Ausführungsbeispiele im Zusammenhang mit den beiliegenden Zeichnungen näher beschrieben und erläutert. Es zeigen:
- Figur 1: eine schematische Ansicht eines erfindungsgemäßen chirurgischen Instruments;
- Figur 2: eine schematische Seitenansicht eines vorderen Abschnitts des chirurgischen Instruments in geschlossenem Zustand;
- Figur 3: eine entsprechende Seitenansicht des chirurgischen Instruments in geöffnetem Zustand;
- Figur 4: eine schematische Seitenansicht des beweglichen Maulteils;
- Figur 5: eine schematische perspektivische Ansicht des beweglichen Maulteils;
- Figur 6: eine schematische Darstellung des beweglichen Maulteils in einer Draufsicht;
- Figur 7: eine schematische perspektivische Ansicht des Hauptteils im Bereich des feststehenden Maulteils;
- Figur 8: eine schematische perspektivische Ansicht des Schiebers;
- Figur 9: eine schematische Seitenansicht des vorderen Abschnitts des erfindungsgemäßen chirurgischen Instruments in geschlossenem Zustand; und
- Figur 10: eine schematische Darstellung eines erfindungsgemäßen Verfahrens.

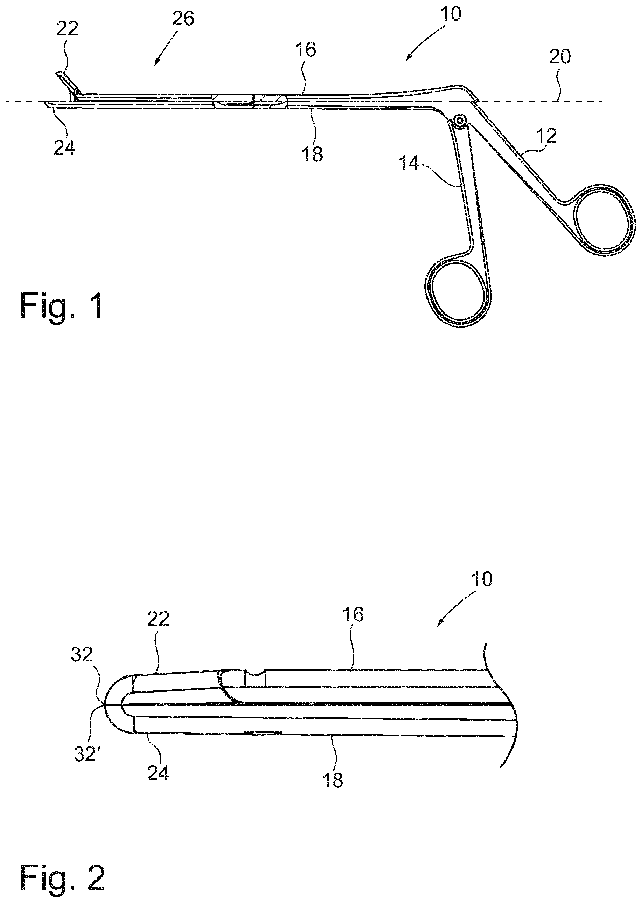

Die Fig. 1 zeigt ein Conchotom bzw. einen Intervertebral Disc (IVD-) Rongeur als beispielhaftes erfindungsgemäßes chirurgisches Instrument 10. Das Instrument wird eingesetzt, um Gewebe zu entfernen. Insbesondere gibt es Anwendungen in der Orthopädie und in der Neurochirurgie, bei denen Knochengewebe an schwer zugänglichen Stellen, beispielsweise im Umfeld der Wirbelsäule, abgetragen werden muss.

Das chirurgische Instrument 10 umfasst einen feststehenden Griff 12 sowie einen beweglichen Griff 14 zum Betätigen des Instruments. Durch den beweglichen Griff 14 wird ein Schieber 16 in Bezug zu einem Hauptteil 18 entlang einer Längsachse 20 des Hauptteils 18 bzw. des chirurgischen Instruments 10 bewegt. Der Schieber 16 und das Hauptteil 18 sind an ein bewegliches Maulteil 22 gekoppelt, durch das das chirurgische Instrument 10 seine Funktion des Greifens und/oder Schneidens von Gewebe erfüllen kann. Durch die Relativbewegung zwischen Schieber 16 und Hauptteil 18 kann das bewegliche Maulteil 22 in Relation zu einem feststehenden Maulteil 24, das mit dem Hauptteil 18 verbunden ist, geöffnet werden. Durch die über den feststehenden Griff 12 und den beweglichen Griff 14 ausgeübte Kraft kann das bewegliche Maulteil 22 wieder in Richtung des feststehenden Maulteils 24 bewegt werden, um das chirurgische Instrument 10 in eine geschlossene Position zu bringen und Gewebe zwischen beweglichem Maulteil 22 und feststehendem Maulteil 24 zu schneiden bzw. zu greifen.

Die vorliegende Erfindung bezieht sich insbesondere auf die Kopplung zwischen Schieber 16, Hauptteil 18 und beweglichem Maulteil 22 in einem vorderen Bereich 26 des chirurgischen Instruments 10. Der dargestellte IVD-Rongeur ein stellt ein Beispiel für ein erfindungsgemäßes chirurgisches Instrument 10 dar. Die Erfindung kann jedoch auch bei anderen chirurgischen Instrumenten, bei denen ebenfalls eine Bewegungskopplung verwendet wird, eingesetzt werden.

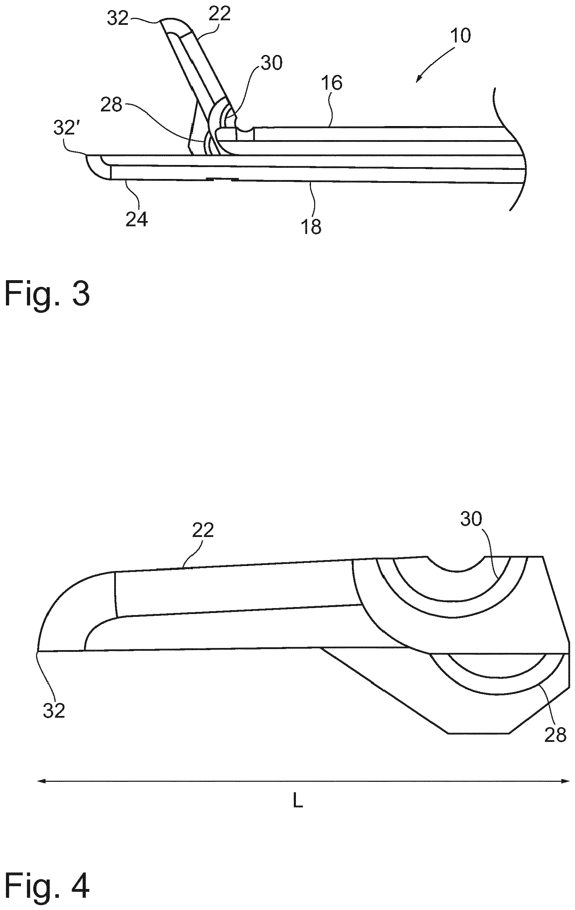

In den folgenden Figuren wird die Funktion des erfindungsgemäßen chirurgischen Instruments 10 beschrieben. Zur besseren Übersichtlichkeit ist in den Figuren 2 und 3 insbesondere der vordere Bereich 26 des chirurgischen Instruments 10 dargestellt.

Die Fig. 2 zeigt das chirurgische Instrument 10 in seinem vorderen Bereich in geschlossener Position. Die Fig. 3 zeigt das erfindungsgemäße chirurgische Instrument 10 in seinem vorderen Bereich in geöffneter Position. Schieber 16 und Hauptteil sind 18 sind zueinander verschieblich gekoppelt, beispielsweise in Form einer Schienenführung. Wie dargestellt bewirkt ein Verschieben des Schiebers 16 gegenüber dem Hauptteil 18 weg vom Arbeitsende (nach rechts in der Darstellung der Figs. 2 und 3) des chirurgischen Instruments 10 ein Öffnen des beweglichen Maulteils 22 in Relation zu dem feststehenden Maulteil 24. Das bewegliche Maulteil 22 umfasst an seiner Vorderseite eine Schnittkante 32, die es erlaubt, beim Schließen Gewebe abzutragen. Auch das feststehende Maulteil umfasst eine Schnittkante 32'.

Um die lineare Bewegung des Schiebers 16 in Bezug zu dem Hauptteil 18 in die Drehbewegung des beweglichen Maulteils 22 in Bezug zu dem Schieber 16 und zu dem Hauptteil 18 umzusetzen, umfasst das bewegliche Maulteil 22 zwei Steuerungselemente, die mit Führungselementen am Schieber 16 bzw. am Hauptteil 18 zusammenwirken. Ein erstes bogenförmiges Steuerungselement 28 wirkt mit einem ersten bogenförmigen Führungselement (in der Darstellung nicht sichtbar) am Hauptteil 18 in einer Kulissensteuerung zusammen. Ein zweites bogenförmiges Steuerungselement 30 wirkt in entsprechender Weise mit einem zweiten bogenförmigen Führungselement (in der Darstellung nicht sichtbar) am Schieber 16 zusammen. Unter einer Kulissensteuerung wird hierbei eine Zwangsführung verstanden, bei der ein Vorsprung an einem Element in einer Nut am anderen Element geführt wird. Durch die dargestellten bogenförmig verlaufenden Führungen der Steuerungselemente 28, 30 wird ein Öffnen des beweglichen Maulteils 22 bewirkt. Eine Kulissensteuerung entspricht insoweit einem Kurvengelenk, bei dem eine gekurvte Flanke durch Gleiten und/oder Wälzen geführt wird. Das bewegliche Maulteil 22 kann sich nur in der durch die Kulissensteuerung vorgegebenen Bewegungsrichtung bewegen.

Dadurch, dass sowohl die Kopplung zwischen beweglichem Maulteil 22 und Schieber 16 als auch die Kopplung zwischen beweglichem Maulteil 22 und Hauptteil 18 über eine Kulissensteuerung realisiert ist, ergibt sich eine hohe Stabilität. Es wird möglich, hohe Kräfte auch bei vergleichsweise filigraner Bauweise aufzunehmen. Aufgrund dessen, dass auf allen Seiten der bogenförmigen Führungs- bzw. Steuerungselemente eine relativ hohe Materialdicke vorliegt, wird ein Ausbrechen vermieden.

In der Fig. 4 ist eine vergrößerte Ansicht des beweglichen Maulteils 22 dargestellt. Wie dargestellt sind beide bogenförmigen Steuerungselemente 28, 30 in dieselbe Richtung gekrümmt, weisen insoweit also dieselbe Krümmungsrichtung auf. Beide Steuerungselemente 28, 30 sowie die korrespondierenden Führungselemente sind als Bogen mit identischer Krümmungsrichtung ausgebildet. Wenn eine Projektion der dargestellten Steuerungselemente 28, 30 in die Darstellungsebene betrachtet wird, kann von der Schnittkante 32 aus betrachtet für beide Steuerungselemente 28, 30 eine Linkskurve bzw. ein Bogen mit positiver Krümmung definiert werden. Relevant ist, dass beide Steuerungselemente 28, 30 (sowie die korrespondierenden Führungselemente) in dieselbe Richtung gekrümmt sind. Je nach Definition und Betrachtungsweise weisen demnach beide Steuerungselemente 28, 30 sowie die korrespondierenden Führungselemente eine positive bzw. negative Krümmung auf.

Wie weiterhin dargestellt, sind das erste Steuerungselement 28 sowie das zweite Steuerungselement 30 im dargestellten Ausführungsbeispiel kreisbogenförmig ausgebildet. Beide weisen einen Kreisbogen mit einem Mittelpunktswinkel von ca. 150° auf. Durch die Verwendung eines derart großen Winkels wird erreicht, dass auch bei einer weiten Öffnung des beweglichen Maulteils 22 in Relation zu dem feststehenden Maulteil eine ausreichende Überdeckung zwischen den Steuerungselementen 28, 30 am beweglichen Maulteil 22 und den Führungselementen am Schieber bzw. am Hauptteil vorliegt. So kann beispielsweise in der geöffneten Position des chirurgischen Instruments ein Öffnungswinkel von 45° bis 55° zwischen beweglichem Maulteil 22 und feststehendem Maulteil erreicht werden und trotzdem die erforderliche Stabilität gewährleistet bleiben.

In der Darstellung ist erkennbar, dass ein gedachter Drehpunkt der Drehbewegung des beweglichen Maulteils 22 gegenüber dem Schieber, der durch den Mittelpunkt des Kreisbogens des zweiten bogenförmigen Steuerungselements 30 definiert ist, außerhalb des beweglichen Maulteils 22 liegt. Ein Mittelpunkt des Kreisbogens des ersten bogenförmigen Steuerungselements 28, der den Drehpunkt der Drehbewegung des beweglichen Maulteils gegenüber dem Hauptteil definiert, liegt innerhalb des beweglichen Maulteils 22. Dadurch, dass aufgrund der gleichgerichteten Krümmungsrichtung der beiden Steuerungselemente 28, 30 vergleichsweise nah beieinanderliegende Drehpunkte der Drehbewegungen des beweglichen Maulteils 22 gegenüber dem Schieber und dem Hauptteil realisiert werden können, kann ein relativ großer Öffnungswinkel des beweglichen Maulteils erreicht werden.

Das dargestellte bewegliche Maulteil 22 ist ein Frästeil, das in einem Fräsprozess hergestellt werden kann. Eine Länge L des beweglichen Maulteils 22 ist vergleichsweise kurz, da aufgrund der gleichen Krümmungsrichtung der beiden Steuerungselemente und der beiden Führungselemente ein großer Bewegungsumfang in relativ geringem Bauraum realisiert werden kann.

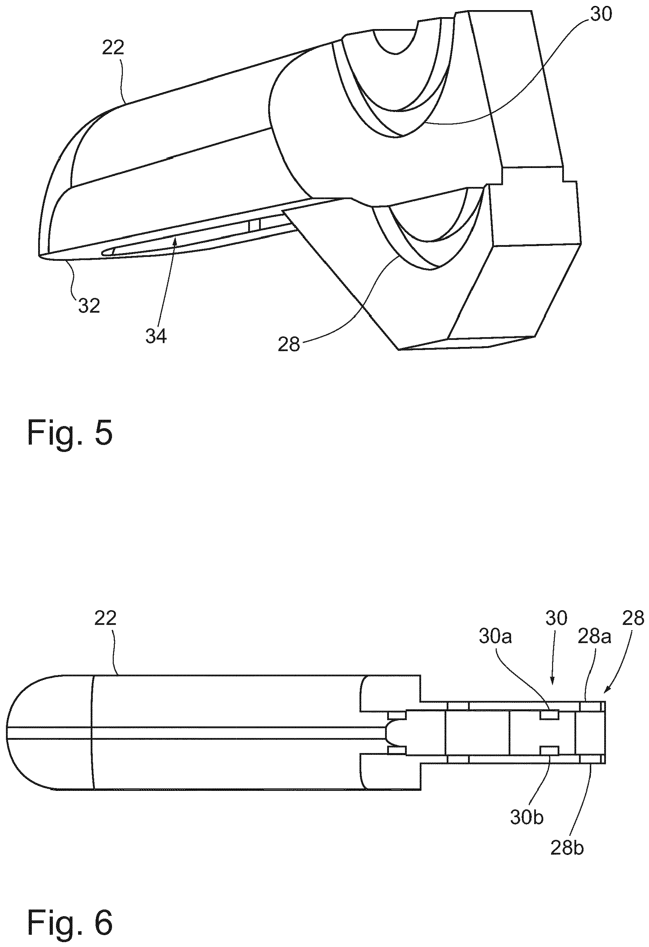

In der Fig. 5 ist eine schematische perspektivische Ansicht des beweglichen Maulteils 22 dargestellt. Es ist ersichtlich, dass sowohl das erste Steuerungselement 28 als auch das zweite Steuerungselement 30 als Nuten, vorzugsweise mit rechteckigem Querschnitt, ausgebildet sind. In die Nuten kann jeweils ein entsprechend ausgebildeter Vorsprung eingreifen. Es versteht sich, dass die Führungselemente am Schieber und am Hauptteil in diesem Fall entsprechend als Vorsprünge mit gleichem bzw. minimal kleinerem Querschnitt ausgebildet sind, um mit den Nuten der Steuerungselemente 28, 30 zu korrespondieren.

Wie dargestellt, umfasst das bewegliche Maulteil 22 eine obere Aushöhlung 34 bzw. Vertiefung und ist insoweit löffelartig ausgebildet. Durch die obere Aushöhlung 34 kann Gewebe aufgenommen werden. Zudem ergibt sich eine verbesserte Anwendung der Schnittkante 32.

In der Fig. 6 ist eine schematische Darstellung des beweglichen Maulteils 22 in einer Draufsicht gezeigt. Im dargestellten Ausführungsbeispiel umfasst das erste bogenförmige Steuerungselement 28 zwei bogenförmige erste Teil-Steuerungselemente 28a, 28b, die beidseitig des beweglichen Maulteils 22 angeordnet sind und auf beiden Seiten mit entsprechenden ersten Teil-Führungselementen am Hauptteil korrespondieren. Analog umfasst das zweite bogenförmige Steuerungselement 30 zwei bogenförmige zweite Teil-Steuerungselemente 30a, 30b, die mit entsprechenden zweiten Teil-Führungselementen am Schieber korrespondieren. Dadurch, dass für das erste Steuerungselement 28 und das zweite Steuerungselement 30 jeweils beidseitig angeordnete symmetrische Teil-Steuerungselemente vorgesehen sind, kann eine verbesserte Kraftverteilung und eine entsprechend verringerte Dimensionierung erfolgen.

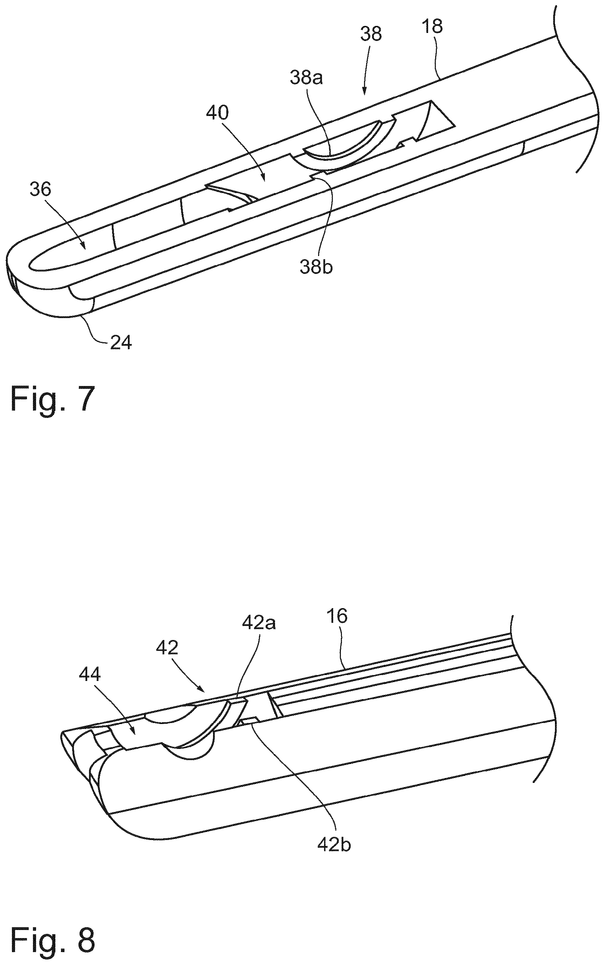

In der Fig. 7 ist eine schematische perspektivische Ansicht des Hauptteils 18 und des feststehenden Maulteils 24 dargestellt. Im dargestellten Ausführungsbeispiel ist auch das feststehende Maulteil 24 löffelförmig ausgebildet und weist eine untere Aushöhlung 36 auf. Das Hauptteil 18 umfasst ein erstes bogenförmiges Führungselement 38, das im dargestellten Ausführungsbeispiel von einem ersten bogenförmigen Teil-Führungselement 38a sowie einem zweiten bogenförmigen Teil-Führungselement 38b gebildet wird. Die beiden Teil-Führungselemente 38a, 38b sind beidseitig symmetrisch an Innenseiten eines Ausschnitts 40 im Hauptteil 18 angeordnet. Das erste Führungselement 38 bzw. die Teilführungselemente 38a, 38b sind korrespondierend zu den zuvor gezeigten Steuerungselementen am beweglichen Maulteil als Vorsprünge ausgebildet. Die Vorsprünge weisen einen Querschnitt auf, der mit dem Querschnitt der Steuerungselemente am beweglichen Maulteil korrespondiert. Auch das Hauptteil 18 ist als Frästeil und einstückig ausgebildet. Der Ausschnitt 40 im Hauptteil 18 ist dazu ausgebildet, das bewegliche Maulteil aufzunehmen.

In der Fig. 8 ist schematisch eine perspektivische Ansicht des Schiebers 16 gezeigt. Das zweite bogenförmige Führungselement 42 am Schieber 16 ist vergleichbar zum ersten Führungselement am Hauptteil ebenfalls zweiteilig ausgebildet und umfasst zwei bogenförmige zweite Teil-Führungselemente 42a, 42b. Auch die zwei bogenförmigen zweiten Teil-Führungselemente 42a, 42b sind in einem Ausschnitt 44 im Schieber 16 angeordnet. Auch der Schieber 16 ist als einstückiges Frästeil ausgeführt, um eine einfache Herstellbarkeit zu ermöglichen. Der Ausschnitt 44 im Schieber 16 ist in Richtung des Hauptteils durchgängig, sodass das bewegliche Maulteil aufgenommen werden kann.

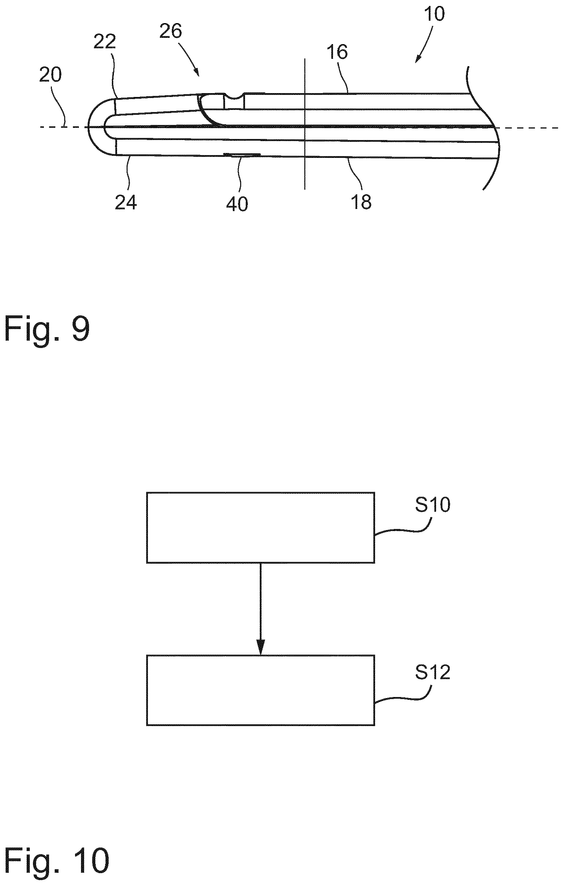

In der Fig. 9 ist schematisch eine Seitenansicht des chirurgischen Instruments 10 in seinem vorderen Bereich 26 dargestellt. In der dargestellten vorteilhaften Ausführungsform verringert sich eine maximale Ausdehnung des Hauptteils, des Schiebers und des beweglichen Maulteils in der geschlossenen Position des chirurgischen Instruments 10 in einer Ebene senkrecht zur Längsachse 20 des Hauptteils 18 in Richtung des feststehenden Maulteils 24. In anderen Worten nimmt ein Querschnitt des chirurgischen Instruments in seinem vorderen Teil in Richtung der Maulteile ab. Hierdurch wird ein verbessertes Verhalten des Instruments beim Verlassen des Operationsbereichs erreicht. Die Gefahr von Verletzungen des umgebenden Gewebes beim Einführen und Herausziehen des chirurgischen Instruments in der geschlossenen Position wird vermindert.

Zudem ist in der Fig. 9 erkennbar, dass der untere Ausschnitt 40 durchgängig ist, sodass das bewegliche Maulteil in der geschlossenen Position bis an die Unterkante des Hauptteils 18 geführt werden kann. Durch den durchgängigen unteren Ausschnitt 40 wird erreicht, dass bei minimal erforderlichem Bauraum ein möglichst stabiles bewegliches Maulteil 22 verwendet werden kann, bei dem vergleichsweise viel Material im Bereich der Steuerungselemente, insbesondere im Bereich des ersten Steuerungselements, vorgesehen ist.

In der Fig. 10 ist schematisch ein erfindungsgemäßes Verfahren zum Herstellen eines chirurgischen Instruments dargestellt. Das Verfahren umfasst Schritte des Fräsens S10 der Führungselemente in das Hauptteil und in den Schieber sowie des Fräsens S12 der Steuerungselemente in das bewegliche Maulteil. Das Verfahren kann beispielsweise von einem Steuergerät einer CNC-Fräsmaschine ausgeführt werden, mit der ein erfindungsgemäßes chirurgisches Instrument hergestellt wird. Das Verfahren kann hierzu beispielsweise in Software implementiert sein und als Computerprogrammprodukt ausgebildet sein.

Die Erfindung wurde anhand der Zeichnungen und der Beschreibung umfassend beschrieben und erklärt. Die Beschreibung und Erklärung sind als Beispiel und nicht einschränkend zu verstehen. Die Erfindung ist nicht auf die offenbarten Ausführungsformen beschränkt. Andere Ausführungsformen oder Variationen ergeben sich für den Fachmann bei der Verwendung der vorliegenden Erfindung sowie bei einer genauen Analyse der Zeichnungen, der Offenbarung und der nachfolgenden Patentansprüche.

In den Patentansprüchen schließen die Wörter "umfassen" und "mit" nicht das Vorhandensein weiterer Elemente oder Schritte aus. Der undefinierte Artikel "ein" oder "eine" schließt nicht das Vorhandensein einer Mehrzahl aus. Ein einzelnes Element oder eine einzelne Einheit kann die Funktionen mehrerer der in den Patentansprüchen genannten Einheiten ausführen. Die bloße Nennung einiger Maßnahmen in mehreren verschiedenen abhängigen Patentansprüchen ist nicht dahingehend zu verstehen, dass eine Kombination dieser Maßnahmen nicht ebenfalls vorteilhaft verwendet werden kann. Ein Computerprogramm kann auf einem nichtflüchtigen Datenträger gespeichert/vertrieben werden, beispielsweise auf einem optischen Speicher oder auf einem Halbleiterlaufwerk (SSD). Ein Computerprogramm kann zusammen mit Hardware und/oder als Teil einer Hardware vertrieben werden, beispielsweise mittels des Internets oder mittels drahtgebundener oder drahtloser Kommunikationssysteme. Bezugszeichen in den Patentansprüchen sind nicht einschränkend zu verstehen.

### Bezugszeichenliste

- 10: chirurgisches Instrument
- 12: feststehender Griff
- 14: beweglicher Griff
- 16: Schieber
- 18: Hauptteil
- 20: Längsachse
- 22: bewegliches Maulteil
- 24: feststehendes Maulteil
- 26: vorderer Bereich
- 28: erstes Steuerungselement
- 30: zweites Steuerungselement
- 32, 32': Schnittkanten am beweglichen bzw. feststehenden Maulteil
- 34: obere Aushöhlung
- 36: untere Aushöhlung
- 38: erstes Führungselement
- 40: Ausschnitt im Hauptteil
- 42: zweites Führungselement
- 44: Ausschnitt im Schieber

## Patentansprüche

1. Chirurgisches Instrument (10) mit:
einem Hauptteil (18) mit einem feststehenden Maulteil (24) an einem Ende;
einem Schieber (16), der relativ zu dem Hauptteil entlang einer Längsachse (20) verschiebbar gelagert ist; und
einem beweglichen Maulteil (22) zum Ausführen einer Drehbewegung gegenüber dem feststehenden Maulteil und dem Schieber zwischen einer geöffneten Position und einer geschlossenen Position, um mit dem feststehenden Maulteil zum Greifen und/oder Schneiden von Gewebe zusammenzuwirken, wobei
das Hauptteil ein erstes bogenförmiges Führungselement (38) umfasst und der Schieber ein zweites bogenförmiges Führungselement (42) umfasst;
das bewegliche Maulteil zum Ausführen der Drehbewegung ein erstes bogenförmiges Steuerungselement (28) umfasst, das mit dem ersten Führungselement in einer Kulissensteuerung zusammenwirkt, und ein zweites bogenförmiges Steuerungselement (30) umfasst, das mit dem zweiten Führungselement in einer Kulissensteuerung zusammenwirkt; und
eine Krümmungsrichtung des ersten Führungselements einer Krümmungsrichtung des zweiten Führungselements entspricht.

2. Chirurgisches Instrument (10) nach Anspruch 1, wobei
das erste Führungselement (38) einen bogenförmigen Vorsprung umfasst und das erste Steuerungselement (28) eine bogenförmige Nut, in die der Vorsprung eingreift, umfasst; und/oder
das zweite Führungselement (42) einen bogenförmigen Vorsprung umfasst und das zweite Steuerungselement (30) eine bogenförmige Nut, in die der Vorsprung eingreift, umfasst.

3. Chirurgisches Instrument (10) nach einem der vorstehenden Ansprüche, wobei
das erste Führungselement (38) und das erste Steuerungselement (28) kreisbogenförmig mit identischem Radius ausgebildet sind; und/oder
das zweite Führungselement (42) und das zweite Steuerungselement (30) kreisbogenförmig mit identischem Radius ausgebildet sind.

4. Chirurgisches Instrument (10) nach Anspruch 3, wobei ein Kreisbogenradius des ersten Führungselements (38) einem Kreisbogenradius des zweiten Führungselements (42) entspricht.

5. Chirurgisches Instrument (10) nach einem der Ansprüche 3 bis 4, wobei
ein Mittelpunktswinkel eines Kreisbogens des ersten Führungselements (38) einem Mittelpunktswinkel eines Kreisbogens des ersten Steuerungselements (28) entspricht; und/oder
ein Mittelpunktswinkel eines Kreisbogens des zweiten Führungselements (42) einem Mittelpunktswinkel eines Kreisbogens des zweiten Steuerungselements (30) entspricht.

6. Chirurgisches Instrument (10) nach einem der Ansprüche 3 bis 5, wobei
ein Mittelpunktswinkel eines Kreisbogens des ersten Führungselements (38) zwischen 160° und 180° liegt; und/oder
ein Mittelpunktswinkel eines Kreisbogens des zweiten Führungselements (42) zwischen 160° und 180° liegt.

7. Chirurgisches Instrument (10) nach einem der vorstehenden Ansprüche, wobei
das erste Steuerungselement (28) zwei bogenförmige erste Teil-Steuerungselemente (28a, 28b) umfasst, die bezüglich einer durch die Längsachse (20) des Hauptteils (18) verlaufenden Bewegungsebene des beweglichen Maulteils (22) symmetrisch auf zwei Außenseiten des beweglichen Maulteils angeordnet sind und mit zwei bogenförmigen ersten Teil-Führungselementen (38a, 38b) des ersten Führungselements (38) an Innenseiten eines Ausschnitts (40) im Hauptteil zusammenwirken; und/oder
das zweite Steuerungselement (30) zwei bogenförmige zweite Teil-Steuerungselemente (30a, 30b) umfasst, die bezüglich einer durch die Längsachse (20) des Hauptteils verlaufenden Bewegungsebene des beweglichen Maulteils symmetrisch auf zwei Außenseiten des beweglichen Maulteils angeordnet sind und mit zwei bogenförmigen zweiten Teil-Führungselementen (42a, 42b) des zweiten Führungselements (42) an Innenseiten eines Ausschnitts (44) im Schieber (16) zusammenwirken.

8. Chirurgisches Instrument (10) nach einem der vorstehenden Ansprüche, wobei
das bewegliche Maulteil (22) und die beiden Steuerungselemente (28, 30) einstückig als Frästeil ausgebildet sind;
das Hauptteil (18) und das erste Führungselement (38) einstückig als Frästeil ausgebildet sind; und/oder
der Schieber (16) und das zweite Führungselement (42) einstückig als Frästeil ausgebildet sind.

9. Chirurgisches Instrument (10) nach einem der vorstehenden Ansprüche, wobei das bewegliche Maulteil (22) und/oder das feststehenden Maulteil (24) löffelförmig ausgebildet sind.

10. Chirurgisches Instrument (10) nach einem der vorstehenden Ansprüche, wobei sich ein Querschnitt des Hauptteils (18), des Schiebers (16) und des beweglichen Maulteils (22) senkrecht zu der Längsachse (20) des Hauptteils in Richtung des feststehenden Maulteils (24) verringert.

11. Chirurgisches Instrument (10) nach einem der vorstehenden Ansprüche, wobei ein Drehpunkt der Drehbewegung des beweglichen Maulteils (22) gegenüber dem Schieber (16) außerhalb des beweglichen Maulteils liegt.

12. Chirurgisches Instrument (10) nach einem der vorstehenden Ansprüche, wobei ein Drehpunkt der Drehbewegung des beweglichen Maulteils (22) gegenüber dem feststehenden Maulteil (24) innerhalb des beweglichen Maulteils liegt.

13. Chirurgisches Instrument (10) nach einem der vorstehenden Ansprüche, wobei die geöffnete Position des beweglichen Maulteils (22) einen Öffnungswinkel von 45° bis 55° aufweist.

14. Chirurgisches Instrument (10) nach einem der vorstehenden Ansprüche, wobei das bewegliche Maulteil (22) und das feststehende Maulteil (24) aus Edelstahl ausgebildet sind.

15. Verfahren zum Herstellen eines chirurgischen Instruments (10), mit einem Hauptteil (18) mit einem feststehenden Maulteil (24) an einem Ende; einem Schieber (16), der relativ zu dem Hauptteil entlang einer Längsachse (20) verschiebbar gelagert ist; und einem beweglichen Maulteil (22) zum Ausführen einer Drehbewegung gegenüber dem feststehenden Maulteil und dem Schieber zwischen einer geöffneten Position und einer geschlossenen Position, um mit dem feststehenden Maulteil zum Greifen und/oder Schneiden von Gewebe zusammenzuwirken, mit den Schritten:
Fräsen (S10) eines ersten bogenförmigen Führungselements (38) in das Hauptteil und eines zweiten bogenförmigen Führungselements (42) in den Schieber;
Fräsen (S12) eines ersten bogenförmigen Steuerungselements (28) zum Ausführen der Drehbewegung in das bewegliche Maulteil, das mit dem ersten Führungselement in einer Kulissensteuerung zusammenwirkt, und eines zweiten bogenförmigen Steuerungselements (30) in das bewegliche Maulteil, das mit dem zweiten Führungselement in einer Kulissensteuerung zusammenwirkt, wobei
eine Krümmungsrichtung des ersten Führungselements einer Krümmungsrichtung des zweiten Führungselements entspricht.
